# EUROPEAN PATENT APPLICATION

(11) **EP 2 559 774 A1**
(43) Date of publication of application: **20.02.2013**
(21) Application number: 12180072.6
(22) Date of filing: 10.08.2012
(51) Int. Cl.: C12Q 1/68

(54) **Improved method for amplification of target nucleic acids using a multi-primer approach**

(30) Priority: 17.08.2011 EP 11177735
(71) Applicant: Roche Diagnostics GmbH, 68305 Mannheim (DE); F. Hoffmann-La Roche AG, 4070 Basel (CH)
(72) Inventor: Frey, Bruno, Dr., 82377 Penzberg (DE); Labaere, Irene, 82377 Penzberg (DE)
(74) Representative: Burger, Alexander

(57) **Abstract**

The present invention is a method for amplification of target nucleic acids wherein a first and second primer set is used to amplify a target nucleic acid in a single amplification reaction. The primers of the first primer set generate a first amplification product that serves as a template for amplification by the primers of the second primer set due to the incorporation of a first and second tag sequence added to the target nucleic acid from the forward and reverse primers of the first primer set to which the primers of the second primer set can hybridize to its complement. Additional sequences are thereby added to the resulting target nucleic acid amplicons because of further amplification from the first amplification products by the primers of the second primer set.

## Description

### Field of the Invention

The present invention relates generally to the fields of molecular biology and to DNA sequencing of target nucleic acids. More specifically, the invention relates to efficient generation of target nucleic acid amplicons in a single amplification reaction containing multiple primer sets which facilitates further downstream processing of the amplified target nucleic acids.

### Background of the Invention

The Polymerase Chain Reaction (PCR) has become an invaluable and powerful tool within the research and diagnostics communities. At its most basic level, PCR comprises a method for the amplification of target nucleic acids from variable sources of input DNA. Employed in the amplification, as part of a PCR reaction mixture, are primers directed to specifically hybridize and flank known sequences of the input DNA which anneal such that their orientation allows the 5'-3' extension by DNA polymerase of the target nucleic acids. The temperature cycling of PCR using denaturation, annealing and extension steps allows for the hybridization of the free primers in the reaction mixture to the target nucleic acid sequences and thereby facilitates the exponential production of the target nucleic acids by DNA polymerase. Specificity of the amplified target nucleic acid products is directly related to the primer sequences used in the PCR amplification.

Regardless of the engineered specificity from the primers used in PCR, incomplete knowledge of the input DNA's sequence can result in amplification of different regions that are not of interest due to recurring domain sequences and/or paralogous genes. Nested PCR has addressed the issues which arise in PCR when the full sequence of the input DNA to be amplified is unknown and non-specific products result from a given primer set. With nested PCR, a first and second primer set combination is used to amplify a first larger PCR product which includes the target nucleic acid sequence of interest and also the sequence wherein the primers of the second set hybridize to amplify a second smaller region within the product of the first primer set in a subsequent amplification reaction. This results in a reduction of non-specific PCR products because the second, smaller PCR products occur only from amplification of a second sequence found downstream of the first PCR product's priming sites that may have otherwise produced non-specific products if used alone.

Due to its versatility, PCR has been modified to generate many different tests and enabled many technologies. In particular, next-generation sequencing methods use PCR to generate the clonality required to analyze the multiple applications offered by such technology. One such application is that of amplicon sequencing where initial and subsequent amplifications create the target nucleic acid amplicons and the requisite clonality, respectively. With the varied sequencers available on the market, the predominant methods used for achieving the desired clonality in samples are emulsion PCR and bridge amplification.

Those of ordinary skill in the art will appreciate the addition of known adaptor sequences to library fragments to be sequenced in order to be successful in producing the clonality. These adaptor sequences for subsequent amplifications are often platform specific, each synthesized according to their own optimized conditions. Typically, each adapter sequence has at least one primer binding site for clonal amplification and in some cases, an additional sequencing primer site. In still other cases, a target nucleic acid identifier sequence, often referred to as a "barcode" sequence, can be added to one or more of the adaptor sequences for purposes of separating multiple target nucleic acid samples within pooled sample mixtures.

Adaptor sequences, in the context of amplicon sequencing, can be added in a variety of ways to the target nucleic acid molecules generated from a primary PCR reaction. Some approaches known in the art use a ligation reaction with DNA Ligase to attach a known adaptor sequence to the available 5' and 3' terminal ends of each fragment. This can be useful in situations where keeping the primary amplification primer sizes short is beneficial.

Still another approach is by including adaptor sequences within the primary amplification primers, sometimes referred to as fusion primers. One of ordinary skill in the art will recognize that the template specific portion of the fusion primers in this method is at the 3' end, while the additional sequences relevant to the clonal amplification and sequencing are located 5' of the template specific portion. At the beginning of the primary amplification, only the template specific portion of the fusion primer hybridizes to the target nucleic acids. Subsequent rounds of amplification allow the whole length of the fusion primer to anneal to the products. This method is efficient in that there are no further modifications to samples for further processing in a sequencing context, yet the fusion primers can exceed 50 bases in length due to the addition of platform requirements.

Another method is the use of multiple primers within the same primary PCR reaction which amplify the same locus and is similar in setup to Nested PCR, yet different in its application. In the multi-primer approach, a first set of forward and reverse primers comprises a 3' template specific sequence that hybridizes to a target nucleic acid and a 5' tag sequence that has minimal, if any, complementarity to the target nucleic acid to be amplified. The products of the first primer set, upon sufficient rounds of amplification, thereafter provide a template for the second primer set to amplify by virtue of the tag sequence. The 5' tag sequence of the first primer set allows the forward and reverse primers of the second primer set to generate the target nucleic acid amplicons of the first primer set along with additional sequences at their 5' and 3' ends. These longer second amplicon products are therefore separate from the products of the first primer set, which can be used in, but not limited to, applications such as next generation sequencing. Descriptions of the aforementioned applications for one of ordinary skill in the art to reproduce can be obtained in sources such as: Viljoen, et al., Molecualr Diagnostics PCR Handbook, Springer-Vellag NY Inc. (2005); Amplicon Fusion Primer Design Guidelines for GS FLX Titanium Series Lib-A Chemistry, Technical Bulletin, TCB No. 013-2009, August 2009; Saiki, et al., Primer-Direected Enzymatic Amplification of DNA with a Thermostable DNA Polymerase, Science, Vol. 239, 4839 (1988) 487-491; Albert and Fenyo, Jour. Clin. Micro. 28 (1990) 1560-1564; PCT Publication No. WO 2010/115154 A1; PCT Publication No. WO 2009/03625 A2.

The present invention utilizes a multi-primer approach to amplify target nucleic acids with a first and second primer set, yet has embodiments that are advantageous over existing methods which include, but are not limited to: the ability to use first primer set primers at an equivalent concentration to or in excess of the concentration of the second primer set primers to yield the longer, second amplification products; the use of at least partially 3' phosphorylated second primer set primers in amplification; and the use of a polymerase with proofreading ability or a polymerase that is combined with a thermostable exonuclease to produce first and second amplification products.

### Summary of the Invention

The purpose of the present invention is to create a plurality of target nucleic acid amplicons from an amplification mixture comprising multiple primer sets. Each primer set is differentiated by the sequence regions that provide variable functionality based on their role within the scheme. The primers of the first set comprise a sequence which is specific to a region of the target nucleic acid of interest at the primer's 3' end and generates a first amplification product. The primers of the first set also minimally comprise a second sequence at their 5' end which is a sequence that allows for a second primer, ideally those of the second primer set, to prime therefrom and generate a second amplification product.

Primers of the second primer set comprise a sequence identical at their 3' ends to the 5' portion of the primers of the first set to generate the second amplification products. In addition to this sequence, the primers of the second set may also contain additional sequences 5' to the portion identical to the primers of the first set which can be useful for various applications. Examples of such sequences can be, but are not limited to, amplification and sequencing primer sites.

In some embodiments of the present invention, one or more of the primers of the second set may be at least partially modified at the 3' terminal nucleotide with a group that prevents extension off of the modified oligonucleotide primer. This group can be a phosphate, yet can also be other modifications known in the art that can be removed by a polymerase with the proper proofreading activity or by a thermostable exonuclease capable of removing the modification. In yet further embodiments of the present invention, a proofreading polymerase can be used. This polymerase can have inherent proofreading activity or can be in combination with a thermostable exonuclease capable of removing 3' modifications to oligonucleotide primers.

In yet another embodiment, the primers of the first primer set are used in the amplification at a equivalent concentration or in excess of the concentration of the primers of the second set. Certain fold excesses of the first primer set to second primer set are 2.5-, 5- and 10 fold excesses.

### Brief Description of the Drawings

The above and further features will be more clearly appreciated from the following detailed description when taken in conjunction with the accompanying drawings. In the drawings, like reference numerals indicate like structures, elements, or method steps and the left-most digit of a reference numeral indicates the number of the figure in which the references element first appears. All of these conventions, however, are intended to be typical or illustrative, rather than limiting.

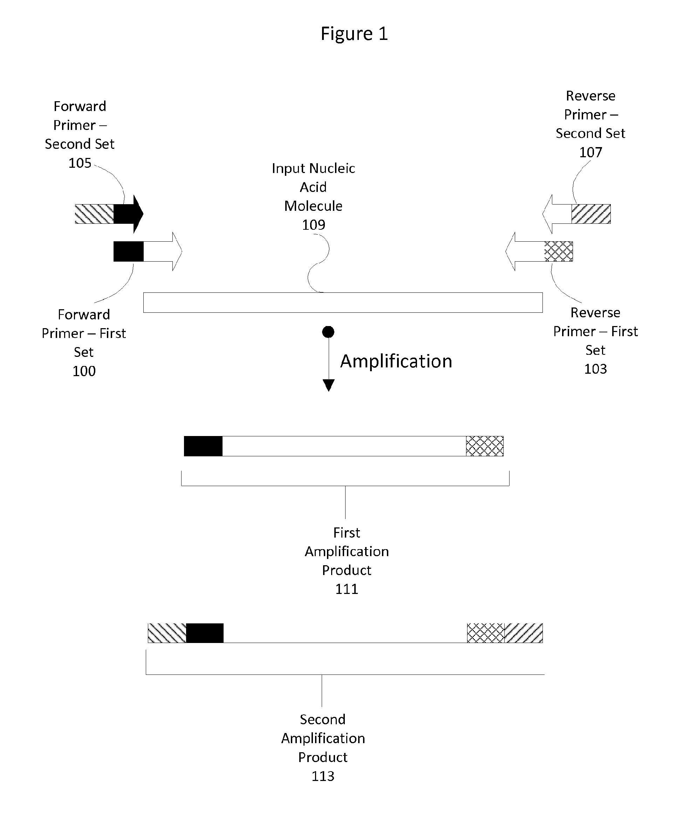

Figure 1 is a simplified graphical representation of embodiments of the amplification products of the method using a first and second primer set to generate a plurality of target nucleic acids containing the sequences of both the first and second primer sets flanking the target nucleic acid sequence;

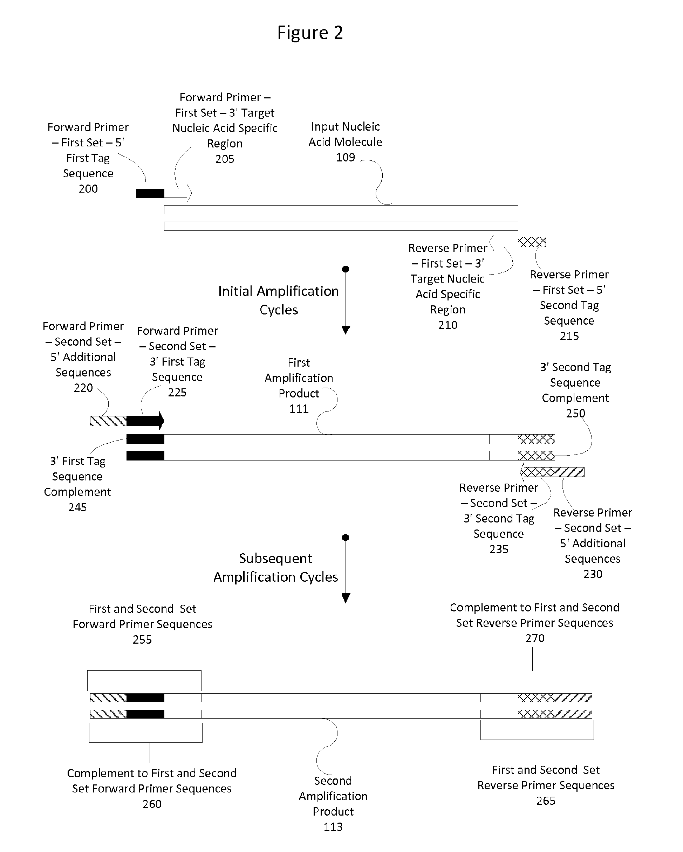

Figure 2 is a more detailed graphical representation of embodiments of the amplification products of the method using a first and second primer set to generate a plurality of target nucleic acids containing the sequences of both the first and second primer sets flanking the target nucleic acid sequence;

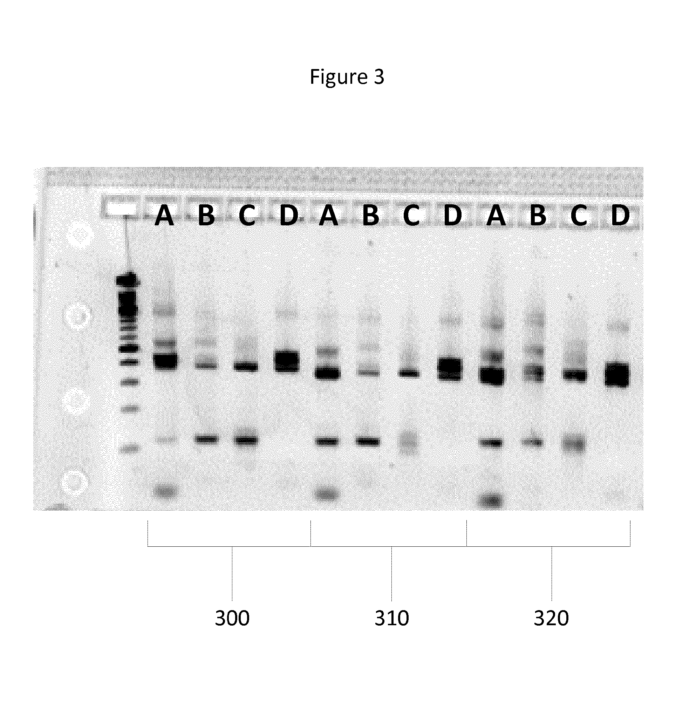

Figure 3 is a photograph of an electrophoresis gel containing the amplification products using the multi-primer scheme with and without the modified 3' termini of the primers of the second primer set, equivalent concentration of the first primer set with respect to the second primer set concentration and also comparing multiple different polymerase systems. The letters A, B, C, and D refer to the RealTime ready DNA Master (unmodified second primer set primers), FastStart High Fidelity Master (unmodified second primer set primers), Expand High Fidelity (unmodified second primer set primers), and Expand High Fidelity (3' modified second primer set primers) amplification systems, respectively.

### Detailed Description of the Invention

As will be described in greater detail below, embodiments of the presently described invention include methods for efficient generation of target nucleic acid amplicons in a single amplification reaction containing multiple primer sets which facilitates further downstream processing of the amplified target nucleic acids.

### A. General

The definitions provided within are provided for clarification in the specification and claims for the detailed explanation and enablement of the present invention. All terms defined within are consistent with the understanding of a person of ordinary skill in the art.

The term "nucleic acid" as used herein generally refers to both DNA or RNA, whether it be a product of amplification, synthetically created, products of reverse transcription of RNA or naturally occuring. Typically, nucleic acids are single- or double-stranded molecules and are composed of naturally occuring nucleotides. Double-stranded nucleic acid molecules can have 3' or 5' overhangs and as such are not required or assumed to be completely double-stranded over their entire length.

Furthermore, the term nucleic acid can be composed of non-naturally occuring nucleotides and/or modifications to naturally occuring nucleotides. Examples are listed herein, but are not limited to: phosphorylation of 5' or 3' nucleotides to allow for ligation or prevention of exonuclease degradation/polymerase extension, respectively; amino, thiol, alkyne, or biotinyl modifications for covalent and near covalent attachments; fluorphores and quenchers; phosphorothioate, methylphosphonates, phosphoroamidates and phosphorotriester linkages between nucleotides to prevent degradation; methylation; and modified bases such as deoxyInosine, 5-Bromo dU, deoxyUridine, 2-Aminopurine, dideoxyCytidine, 5-Methyl dC, locked nucleic acids (LNA's), Iso-dC and -dG bases, 2'-O-Methyl RNA bases and Fluorine Modified Bases.

The term "oligonucleotide" as used herein generally refers to a nucleic acid sequence typically designed to be single-stranded DNA and less than 75 nucleotides in length.

The term "complementary" as used herein generally refers to the ability to form favorable thermodynamic stability and specific pairing between the bases of two nucleotides at an appropriate temperature and ionic buffer conditions. This pairing is dependent on the hydrogen bonding properties of each nucleotide. The most fundamental examples of this are the hydrogen bond pairs between thymine/adenine and cytosine/guanine bases. In the present invention, primers for amplification of target nucleic acids can be both fully complementary over their entire length with a target nucleic acid molecule or "semi-complementary" wherein the primer contains additional, non-complementary sequence minimally capable or incapable of hybridization to the target nucleic acid.

The term "primer" as used herein generally refers to an oligonucleotide that is able to anneal, or hybridize, to a nucleic acid sequence and allow for extension under sufficient conditions (buffer, dNTP's, polymerase, mono- and divalent salts, temperature, etc.) of the nucleic acid to which the primer is complementary.

The term "hybridize" as used herein generally refers to the base-pairing between different nucleic acid molecules consistent with their nucleotide sequences. The terms "hybridize" and "anneal" can be used interchangeably.

The terms "template nucleic acid", "template molecule", "target nucleic acid", or "target molecule" as used herein generally refer to a nucleic acid molecule that is the subject of an amplification reaction that may optionally be interrogated by a sequencing reaction in order to derive its sequence information.

The term "amplification" as used herein generally refers to the production of a plurality of nucleic acid molecules from a target nucleic acid wherein primers hybridize to specific sites on the target nucleic acid molecules in order to provide an inititation site for extension by a polymerase. Amplification can be carried out by any method generally known in the art, such as but not limited to: standard PCR, long PCR, hot start PCR, qPCR, RT-PCR and Isothermal Amplification.

The term "amplicon" as used herein generally refers to selected amplification products which are amplified by a specific set of forward and reverse primers such as those produced from amplification techniques known in the art.

The term "first primer set" as used herein generally refers to the set of primers comprising at least one forward and reverse primer pair which contain a 3' sequence complementary to a target nucleic acid so that efficient amplification of the target nucleic acid may occur and an additional 5' tag sequence to allow further amplification of the resultant first amplification product by the primers of a separate, second primer set.

The term "second primer set" as used herein generally refers to the set of primers comprising a forward and reverse primer which contain a 3' sequence identical to the tag seuqences of the primers of the first set and an additional 5' sequence or sequences which can be used for a variety of purposes. The primers of the second primer set always produce a second amplification product larger than the first amplification product and may include amplification, sequencing, identifier or any other sequences that may be deemed useful.

The term "excess" as used herein generally refers to a larger quantity or concentration of a certain reagent or reagents as compared to another.

The term "equimolar ratio" as used herein generally refers to the ratios of two or more components within a given reaction being in a 1:1 molar ratio with each other. By way of example, an equimolar ratio of forward primer and reverse primer, both of stock concentration 100uM, would be the addition of 1uL forward-and 1uL reverse primer to a given reaction mixture.

The term "first amplification product" as used herein generally refers to the amplification product generated by the primers of the first primer set, wherein the input material for the reaction serves as the template molecule.

The term "second amplification product" as used herein generally refers to the amplification product generated by the primers of the second primer set, wherein the first amplification product generated by the primers of the first primer set serves as the template molecule.

The term "tag sequence" as used herein generally refers to the sequence of the forward and reverse primers of the first primer set at their 5' ends and the sequence of the forward and reverse primers of the second primer set at the 3' ends. This sequence is non-complementary to the target nucleic acids which are the input nucleic acid molecules and also makes possible the second amplification products.

The term "proofreading activity" as used herein generally refers to the ability of some DNA polymerases to correct errors during extension of a nucleic acid molecule. While all DNA polymerases can extend in a 5'-3' direction, proofreading DNA polymerases can also remove bases by a 3'-5' exonuclease activity which removes errors of misincorporation and replaces these errors with the correct nucleotide. Others still have 5'-3' exonuclease activity commonly refered to as nick translation. Description of such polymerases exhibiting this activity may be found in EP 0 930 370 B1.

The term "thermostable exonuclease" as used herein generally refers to the class of enzymes that cleave the terminal phosphodiester bond linking nucleotides within a nucleic acid molecule which is also able to withstand temperatures above 65°C without losing its activity. Description of such enzymes exhibiting this activity may be found in EP 1 277 841 B1.

The terms "template specific region", "target specific region" or "region of interest" as used herein generally refer to the region of a particular nucleic acid molecule that is of scientific interest. These regions typically have at least partially known sequences in order to design primers which flank the region or regions of interest for use in amplification reactions and thereby recover target nucleic acid amplicons containing these regions of interest.

The term "qPCR" as used herein generally refers to the PCR technique known as real-time quantitative polymerase chain reaction, quantitative polymerase chain reaction or kinetic polymerase chain reaction. This technique simultaneously amplifies and quantifies target nucleic acids using PCR wherein the quantification is by virtue of an intercalating fluorescent dye or sequence-specific probes which contain fluorescent reporter molecules that are only detectable once hybridized to a target nucleic acid.

### B. Embodiments of the Presently Described Invention

The present invention describes a method for amplifying target nucleic acids by providing a first primer set with a forward and reverse primer such that each primer contains a target specific region capable of hybridizing to a target nucleic acid, wherein the forward primer comprises a first tag sequence at its 5' end and the reverse primer comprises a second tag sequence at its 5' end. The second primer set comprises a forward primer with a 3' region identical to the first tag sequence at the 5' end of the forward primer of the first primer set and a reverse primer with a 3' region identical to the second tag sequence at the 5' end of the reverse primer of the first primer set. These primer sets are then used to amplify the target nucleic acids in the presence of the first and second primer sets wherein the primers of the first primer set are added to the amplification mixture and amplified by a proofreading polymerase or combined enzyme mixture comprising a polymerase lacking a 3'-5' exonuclease activity (proofreading) and 3'-5' thermostable exonuclease and thereby amplify multiple target nucleic acids.

Figures 1 and 2 describe graphically the products generated by the first and second primer sets when used in a single amplification reaction simultaneously combining the primers of the first and second primer sets, nucleotides, polymerase, buffer and the required mono- and/or divalent salts. Figure 1 illustrates the multi-primer scheme which initially generates a first amplification product 111 from the input molecule 109 by primers 100 and 103 and also the second amplification product 113 from primers 105 and 107 using the first amplification product 111 as the template; the second amplification product 113 being the target nucleic acid amplicon.

Figure 2 illustrates in more detail the basic scheme described in Figure 1. One of ordinary skill in the art will recognize that the input nucleic acid molecule 109 is depicted as a double-stranded nucleic acid molecule, but could just as easily be a single-stranded template molecule and still produce the first and second amplification products (111 and 113, respectively).

In embodiments of the invention, the forward primer of the first primer set 100 contains at least two regions: the 5' First Tag Sequence 200 and the 3' Target Nucleic Acid Specific Region 205. Additionally, the reverse primer of the first primer set 103 also contains at least two regions: the 5' Second Tag Sequence 215 and the 3' Target Nucleic Acid Specific Region 210. The significance of the target nucleic acid specific regions 205 and 210 should be readily apparent to one skilled in the art as necessary in order to amplify a given target nucleic acid of interest and yield a first amplification product 111 which contains the target nucleic acid sequence as well as the first and second tag sequences (200 and 215, respectively).

The first and second tag sequences (200 and 215, respectively) are necessary in order to produce the second amplification products 113 by using the primers of the second primer set (105 and 107, respectively). In embodiments, the forward and reverse primers of the primers of the second primer set hybridize with the first and second tag sequence complements (245, 250) and provide sites which allow a polymerase to extend and generate the second amplification product 113. The second amplification product 113 comprises the total modified target nucleic acid amplicon which contains all sequences engineered into the primers of the first and second primer sets.

Embodiments of the first and second primer sets can include, but are not limited to: sequences which hybridize to input nucleic acid molecules, tag sequences which become part of an amplified nucleic acid molecule and become a site for additional primers to hybridize, identification sequences to separate between products or samples or both, a key sequence, amplification priming sites, sequencing primer sites, nucleotide modifications either 5' or 3' and modified bases.

Embodiments of the described invention include the following primer species designed to produce amplicons containing the aforementioned characteristics:

**Table 1: First Primer Set Forward Primer Sequences**

| | | | |
|---|---|---|---|
| **First Primer Set - Forward** | | | |

| **Seq ID No.** | **Primer Sequence 5' → 3'** | **Primer Length** | **Tm °C** |
|---|---|---|---|
| 1 | GTTGTAAAACGACGGCCAGTATTCAACTAGAGGGCAGCCTTG | 42 | 61 |
| 2 | GTTGTAAAACGACGGCCAGTGAATACCCTGTATGAAGGGAAGC | 43 | 60 |
| 3 | GTTGTAAAACGACGGCCAGTTGTAGCCCAAGAAAATGCAG | 40 | 59 |
| 4 | GTTGTAAAACGACGGCCAGTCATCTCACATAAATGCCATTAACA | 44 | 59 |
| 5 | GTTGTAAAACGACGGCCAGTGAAAATAACATCCAGGGAACCA | 42 | 60 |
| 6 | GTTGTAAAACGACGGCCAGTGGAGTTTTAGAAGAACACCACCA | 43 | 60 |
| 7 | GTTGTAAAACGACGGCCAGTCCAATTTTTGGTAGCAGTGGA | 41 | 60 |
| 8 | GTTGTAAAACGACGGCCAGTTGACCTCCAAACAATACACTGG | 42 | 60 |
| 9 | GTTGTAAAACGACGGCCAGTCCCAGTGTTGAAACAGCA | 38 | 57 |
| 10 | GTTGTAAAACGACGGCCAGTTGGTGAAAATCAGTATTCAAAATCA | 45 | 60 |
| 11 | GTTGTAAAACGACGGCCAGTCTTCTTCACAGGTGCTTTCAAG | 42 | 60 |
| 12 | GTTGTAAAACGACGGCCAGTTTGCCATAGTCAGATGCACAG | 41 | 60 |
| 13 | GTTGTAAAACGACGGCCAGTTTGACTAGACAAACCACTGCTG | 42 | 58 |
| 14 | GTTGTAAAACGACGGCCAGTGGGGTTAAGCTTTGTGGATG | 40 | 60 |
| 15 | GTTGTAAAACGACGGCCAGTAAACCGTTCATTTCTCAGGATG | 42 | 60 |
| 16 | GTTGTAAAACGACGGCCAGTTGCAAGTGACCCTTGTTTTG | 40 | 60 |
| 17 | GTTGTAAAACGACGGCCAGTATAGACACCTATAATATCAGCTGCAC | 46 | 57 |
| 18 | GTTGTAAAACGACGGCCAGTCCATATATTGTGTTTGGGATTCAA | 44 | 60 |
| 19 | GTTGTAAAACGACGGCCAGTTGCTCTATTTTGTGTCATTCCATT | 44 | 60 |
| 20 | GTTGTAAAACGACGGCCAGTGGGACCTGTAGTTGAGGCTGT | 41 | 61 |
| 21 | GTTGTAAAACGACGGCCAGTGAGTTTGGGAGTGTGGAAGC | 40 | 60 |
| 22 | GTTGTAAAACGACGGCCAGTGCCTTCATAAAATAATCATCAACA | 44 | 57 |
| 23 | GTTGTAAAACGACGGCCAGTCCAATCCAGTTAGTCCTTATCCA | 43 | 59 |
| 24 | GTTGTAAAACGACGGCCAGTGCTCAGTCTACCACCCATCC | 40 | 60 |
| 25 | GTTGTAAAACGACGGCCAGTCATTCACCTTCTCACATAATCCA | 43 | 59 |
| 26 | GTTGTAAAACGACGGCCAGTAGACAGCGAGCAGAGCTTTC | 40 | 60 |
| 27 | GTTGTAAAACGACGGCCAGTAGCCCGTGAGAAAGAGGAAG | 40 | 61 |
| 28 | GTTGTAAAACGACGGCCAGTCCAGACTAGATGCTTTCTGGTTTA | 44 | 59 |
| 29 | GTTGTAAAACGACGGCCAGTGATGCATCTGTTACTATCTTTTGC | 44 | 57 |
| 30 | GTTGTAAAACGACGGCCAGTGGTACTGGTGGAGTATTTGATAGTG | 45 | 58 |
| 31 | GTTGTAAAACGACGGCCAGTTGTAATAATCCAGACTGTGTTTCTCC | 46 | 60 |

**Table 2: First Primer Set Reverse Primer Sequence**

| **First Primer Set - Reverse** | | | |
|---|---|---|---|
| **Seq ID No.** | **Primer Sequence 5' → 3'** | **Primer Length** | **Tm °C** |
| 32 | CACAGGAAACAGCTATGACCACTGTGCGTTTTATTCCTCCAT | 42 | 60 |
| 33 | CACAGGAAACAGCTATGACCCCCACTGCAGTTATGTGTTGAA | 42 | 61 |
| 34 | CACAGGAAACAGCTATGACCTGGGTGAGTGATCTCACAGG | 40 | 60 |
| 35 | CACAGGAAACAGCTATGACCAGCTTGCAAATTGCTGCTG | 39 | 60 |
| 36 | CACAGGAAACAGCTATGACCCCCTCTATTTTCACTTCCCTTAAA | 44 | 59 |
| 37 | CACAGGAAACAGCTATGACCTCGACCCTTCAGAATCTCTTG | 41 | 59 |
| 38 | CACAGGAAACAGCTATGACCCTTGTGCTCCAGCTGTGTTG | 40 | 61 |
| 39 | CACAGGAAACAGCTATGACCTGAGTTTGAAAATGGCTCAGTC | 42 | 59 |
| 40 | CACAGGAAACAGCTATGACCTTATATTCTGTACTTCCTCCAGTCC | 45 | 57 |
| 41 | CACAGGAAACAGCTATGACCCCCTGTAGAACTGAAGCTTGTTG | 43 | 60 |
| 42 | CACAGGAAACAGCTATGACCATACAGGCATGTGGCTTGC | 39 | 60 |
| 43 | CACAGGAAACAGCTATGACCCTGAAGAAGTTGTTTGCTGCTCT | 43 | 60 |
| 44 | CACAGGAAACAGCTATGACCTTTATGAGCCTTTACAAATTGCTG | 44 | 60 |
| 45 | CACAGGAAACAGCTATGACCTTGTGACTCTCTGGTGAATAGCA | 43 | 60 |
| 46 | CACAGGAAACAGCTATGACCGTAATGTTCTTTTTAACTGGCATGA | 45 | 59 |
| 47 | CACAGGAAACAGCTATGACCAACCAAAGATTGGGCTTTCC | 40 | 60 |
| 48 | CACAGGAAACAGCTATGACCCAGTTTGGGAAAAACTTTGATTA | 43 | 57 |
| 49 | CACAGGAAACAGCTATGACCGCAGTGGTTTCAACAATTAAGAG | 43 | 60 |
| 50 | CACAGGAAACAGCTATGACCCAGTGTGAGAACAGACTCAACAG | 43 | 61 |
| 51 | CACAGGAAACAGCTATGACCGGGGCTGACTTTTCCTTTTC | 40 | 60 |
| 52 | CACAGGAAACAGCTATGACCGGGGGCAAAACCAAAATAAT | 40 | 60 |
| 53 | CACAGGAAACAGCTATGACCCTGCAGCTTGAGATGAGGTG | 40 | 60 |
| 54 | CACAGGAAACAGCTATGACCAAAACTCTGGCTATTTCCAAACC | 43 | 60 |
| 55 | CACAGGAAACAGCTATGACCAGATGCAGGGCATGAAGAGA | 40 | 61 |
| 56 | CACAGGAAACAGCTATGACCGAATTGACCCATGAGTTGGAG | 41 | 59 |
| 57 | CACAGGAAACAGCTATGACCAAGTTTCATGTGGCTCAGCA | 40 | 59 |
| 58 | CACAGGAAACAGCTATGACCACTGTGACCTTTCCCCACTG | 40 | 60 |
| 59 | CACAGGAAACAGCTATGACCCCGAATTTTCCAAGGTTATTACA | 43 | 59 |
| 60 | CACAGGAAACAGCTATGACCCCTATCATTGTAGTTTGGATATCGT | 45 | 58 |
| 61 | CACAGGAAACAGCTATGACCTGAAACCCAAGGTACATTTCAG | 42 | 59 |
| 62 | CACAGGAAACAGCTATGACCCAGGGATATTACCTACCTCATAAACA | 46 | 59 |

In designing primers for use in embodiments of the present invention, consideration should be given to the following parameters that one of ordinary skill in the art would evaluate, such as: the GC content of the primer preferably about 50-60%; the length of the primer since oligonucleotide quality drops each time a base is added during synthesis; melting temperatures between 55-65°C, although they can be increased as high as 80°C; avoiding self-complemenarity of 3' ends to decrease the likelihood of primer-dimers in amplification; avoiding sequences that will prime at sites elsewhere within a target nucleic acid molecule; avoiding the possibility of forming secondary structures such as hairpins; and incorporating a GC clamp at the 3' end of the primer to aid in stronger binding to the template nucleic acid molecule.

**Table 3: Second Primer Set Sequences**

| **Seq ID No.** | **Second Primer Set - 5°→ 3°** | **Primer Length** | **Tm °C** |
|---|---|---|---|
| 63 | **SPS_Forward** | 55 | 73.7 |
| | CGTATCGCCTCCCTCGCGCCATCAGACGAGTGCGT GTTGTAAAACGACGGCCAGT | | |
| 64 | **SPS_Reverse :** | 55 | 73.3 |
| | CTATGCGCCTTGCCAGCCCGCTCAGACGAGTGCGT CACAGGAAACAGCTATGACC | | |

**Table 4: Target Nucleic Acid Amplicons - First and Second Amplification Products**

| **First Primer Set Paired Seq ID No.** | **Length [including First Primer Set Sequence]** | **First Primer Set plus Second Primer Set Multi-Primer Seq ID No.** | **Length [including First and Second Primer Set Sequences]** |
|---|---|---|---|
| 1&32 | 338 | 1&32, 63&64 | 408 |
| 2&33 | 335 | 2&33, 63&64 | 405 |
| 3&34 | 343 | 3&34, 63&64 | 413 |
| 4&35 | 350 | 4&35, 63&64 | 420 |
| 5&36 | 350 | 5&36, 63&64 | 420 |
| 6&37 | 348 | 6&37, 63&64 | 418 |
| 7&38 | 342 | 7&38, 63&64 | 412 |
| 8&39 | 350 | 8&33, 63&64 | 420 |
| 9&40 | 350 | 5&40, 63&64 | 420 |
| 10&41 | 336 | 10&41, 63&64 | 406 |
| 11&42 | 348 | 11&42, 63&64 | 418 |
| 12&43 | 350 | 12&43, 63&64 | 420 |
| 13&44 | 343 | 13&44, 63&64 | 413 |
| 14&45 | 346 | 14&45, 63&64 | 416 |
| 15&46 | 335 | 15&46, 63&64 | 405 |
| 16&47 | 341 | 16&47, 63&64 | 411 |
| 17&48 | 349 | 17&48, 63&64 | 419 |
| 18&49 | 337 | 18&48, 63&64 | 407 |
| 19&50 | 341 | 19&50, 63&64 | 411 |
| 20&51 | 347 | 20&51, 63&64 | 417 |
| 21&52 | 335 | 21&52, 63&64 | 405 |
| 22&53 | 344 | 22&53, 63&64 | 414 |
| 23&54 | 350 | 23&54, 63&64 | 420 |
| 24&55 | 339 | 24&55, 63&64 | 409 |
| 25&56 | 346 | 25&56, 63&64 | 416 |
| 26&57 | 335 | 26&57, 63&64 | 405 |
| 27&58 | 336 | 27&58, 63&64 | 406 |
| 28&59 | 348 | 28&59, 63&64 | 418 |
| 29&60 | 332 | 29&60, 63&64 | 402 |
| 30&61 | 350 | 30&61, 63&64 | 420 |
| 31&62 | 350 | 31&62, 63&64 | 420 |

In an alternative embodiment, it can be seen from Tables 1 and 2 that the primers of the first set all contain identical tag sequences whether they be the first tag sequence or second tag sequence. The first tag and second tag sequence refers to the sequence at the 5' end of the forward and reverse primers of the first primer set which allows the primers of the second primer set to amplify the first amplification product of the primers of the first primer set, thereby creating the target nucleic acid amplicons. The forward primer of the first primer set will be recognized by those of ordinary skill in the art that the primer hybridizes to the complementary sequence of the 5' terminus of the region of interest within the input nucleic acid molecule that is the subject of amplification. Likewise, the reverse primer of the first primer set will be recognized by those of ordinary skill in the art that the primer hybridizes to the sequence of the 3' terminus of the region of interest within the input nucleic acid molecule that is the subject of amplification.

While these sequences in Tables 1 and 2 are all identical within either the forward or reverse primers, respectively, the sequences may be changed to and serve as an additional level of identification between amplicons within a given sample. Care should be taken to design these changes so that they mirror the fashion in which a typical identifier, or "barcode", sequence would be made and thereby avoid any mispriming within an input nucleic acid molecule which would generate non-specific amplification products.

Embodiments of the invention also use a polymerase which has proofreading activity or is used in combination with a thermostable exonuclease. Due to the use of some embodiments of the present invention utilizing modifications at the 3' end of at least one of the primers of the second primer set, a polymerase with proofreading activity is necessary in order to extend from a 3' blocked oligonucleotide primer. In an alternative, a polymerase can be used with no proofreading activity should the primers of the second primer set only be partially modified with a 3' blocking modification or also if the polymerase is used in combination with a 3'-5' thermostable exonuclease capable of cleaving the 3' modifications. It is obvious to one of ordinary skill in the art that thermostability of the exonuclease is necessary in order to withstand the thermocycling temperatures of PCR.

In yet further embodiments of the present invention, at least one of the primers of the second primer set should be at least partially modified at the 3' terminal nucleotide by a phosphate moiety. This modification was used in the present invention due to the availability of polymerases with proofreading activity and also those that are used in combination with a 3'-5' thermostable exonuclease capable of removing the terminal 3' phosphate and is not meant to be limiting.

As will be discussed further in the examples following, it is also possible to use a polymerase without proofreading activity or not in combination with a thermostable exonuclease. It is not completely certain as to why this is the case for some embodiments of the present invention. It is, however, possible that the primer or primers that are 3'-modified do not contain the modification to 100% of the total molecules that are synthesized. This deficiency therefore may be the reason that polymerases without proofreading activity or not in combination with a thermostable exonuclease are able to extend from the un-modified sub-population during amplification.

In still further embodiments, the primers of the first primer set are at least a 5-fold excess of the primers of the second primer set as exemplified by the results of Example 4.

In even further embodiments, when there is a requirement for multiple amplification mixtures to amplify multiple target nucleic acids, the forward primers of the first primer set can comprise identical first tag sequences with each other and the reverse primers of the first primer set can comprise identical second tag sequences. This embodiment employs the identical tag sequences in either or both species of primers in the first primer set in order to have single forward and reverse primers of the second primer set. This is advantageous because it limits the expense in using the present invention and also reduces the complexity.

In yet another embodiment of the present invention, where there is a requirement for multiple amplification mixtures to amplify multiple target nucleic acids, the forward primers of the first primer set can comprise first tag sequences which are different from each other and the reverse primers of the first primer set can comprise second tag sequences which are different from each other. This complexity is useful so that the tag sequences can serve as an additional level of identification of target nucleic acid amplicons within a sample. In many cases, the same target nucleic acid sequence to be amplified can be the subject of interest over different samples which can come from many different sources. Identification between samples can be accomplished by the identifier sequences contained within the primers of the second primer set. Having different tag sequences within the forward and reverse primers of the first primer set allows for target nucleic acid amplicons to be amplified and differentiated within the same sample source. That is, for example, should there be multiple regions of interest within a given genomic DNA source that requires many different forward and reverse primers to amplify those regions, then the tag sequences of each forward and each reverse primer would be different in each case so as to identify which locus within a certain sample the individual target nucleic acid amplicons originate.

Tables 1 and 2 indicate primers of the first set where all forward primer tag sequences and all reverse tag sequences are conserved within their groups. Given these tables containing thirty-one forward and thirty-one reverse primers, one of ordinary skill in the art can appreciate and design, based on proper considerations mentioned and known in the art, thirty-one forward primers comprising different tag sequences and thirty-one reverse primers comprising different tag sequences. These primers would then require thirty-one separate forward primers of the second primer set and thirty-one reverse primers of the second set in order to match those tag sequences of the distinct primers of the first primer set. It is obvious from this scheme that the complexity and the cost to synthesize the oligonucleotide primers increases. These considerations may, however, be outweighed by the advantages of having multiplied identification within and between any given set of samples.

Further embodiments of the present invention comprise an additional sequence in one or both of the primers of the second primer set that is used for identification of the target nucleic acid amplicons. These sequences are generally located 5' of the tag sequence and can be the same or different identification sequence between primers of the second primer set. Using the same sequence is less complex in design; however, greater differentiation can be made from different identification sequences between forward and reverse primers of the second set if that configuration is chosen. By example, a set of twelve identifier sequences which are identical in both the forward and reverse primers of the second primer set would allow for only twelve distinct forward/reverse primer pairs. If used in a manner wherein the identifier sequences are distinct within a forward/reverse primer pair of the second primer set, then up to 132 different permutations are possible. These also can be used in conjunction with those primers wherein the identifier sequence is also identical between the forward and reverse primers of the second primer set and give a possibility for up to 144 permutations for sample differentiation. This second application of the identifier sequences is useful in an application such as sequencing wherein the reads produced from the sequencing run are long enough so that the identifier sequence at the 5' and 3' ends of the sequence read are capable of interrogation.

In still another embodiment, additional sequences of the primers of the second primer set may include a sequence used for an amplification primer site. This sequence is useful in such applications that require further amplification of the target nucleic acid amplicons produced by the described method. In some instances, this may be in such applications that require clonality and use massively parallel amplification techniques to achieve those results, such as, emulsion PCR and bridge amplification.

Still another embodiment, the additional sequence of the primers of the second primer set may comprise a sequence used for a sequencing primer site. This type of primer site is primarily useful in applications where samples are being prepared for further processing by instrumentation capable of generating sequencing read data in order to determine the sequence of individually adapted target nucleic acid amplicons.

In still further embodiments, target nucleic acid amplicons are at least 300 base pairs in length. It should be noted that target nucleic acids will vary in size based on the actual length of the target nucleic acid region of interest. Sizing considerations will also concern the particular mode of sequencing used and what size is most compatible with the clonal amplification process and given output of the platform. Ensuring that the target nucleic acid amplicons are of appropriate size ensures the greatest potential of high quality data in return.

### Examples

### General Experimental Setup

All the experiments were performed with the LightCycler® 480 real time PCR instrument using 96 MWP with human genomic DNA (hgDNA) (Roche, Catalog No.: 11691112001), as the input nucleic acid template. For determination of the amplicon length, gel electrophoresis was performed with E-Gels (2%) from Invitrogen. Images of the Ethidiumbromide stained gels were taken with a LumiImager (Roche).

Optimization was performed using different concentrations of first primer set and second primer set primer pairs as well as different concentrations of hgDNA, different LightCycler® 480 run protocols and different Mg²⁺ concentrations for the PCR reaction. Negative controls were also included.

### Example 1

Initial experiments performed with the first and second primer sets were conducted with the LightCycler® 480 SYBR Green I Master (Roche, Catalog No.: 04707516001, 04887352001) according to prior art. The reaction mixture for the individual amplification reactions is as follows:

| | |
|---|---|
| LightCycler® 480 SYBR Green I Master (2X Stock) | 10uL |
| Human genomic DNA (10ng/uL Stock) | 3uL |
| Seq ID No. 30 (5uM) | 2uL |
| Seq ID No. 61 (5uM) | 2uL |
| Water. PCR Grade | 3uL |
| Total Reaction Volume | 20uL |

PCR Grade Water (Roche, Catalog No.: 03315843001). The SYBR Green I Master system contains Roche FastStart Taq DNA Polymerase (Roche, Catalog No.: 12032902001) which has 5'-3' polymerase activity as well as no 3'-5' exonuclease activity. The following LightCycler® 480 protocol was used for amplifying the human K-RAS gene with the forward and reverse primers of the first and second primer sets (Tables 1, 2 and 3), specifically Seq ID No. 30, 61, 63, 64:

| **Step** | | **Temp.** | **Duration** | **Ramp Rate** |
|---|---|---|---|---|
| Initial FastStart Activation | | 95°C | 10min | 4.4°C/sec |
| Amplification (45 Cycles) | | | | |
| | Denaturation | 95°C | 10sec | 4.4°C/sec |
| | Annealing | 63°C | 30sec | 2.2°C/sec |
| | Extension | 72°C | 25sec | 4.4°C/sec |
| Final Extension | | 72°C | 7min | 4.4°C/sec |

| | | | | |
|---|---|---|---|---|
| *Melting Curve program followed amplification* | | | | |

The LightCycler® 480 protocol used with the above amplification is a touch down protocol and had the following additional settings associated with annealing segment of the Amplification Step:

| **2nd Anneal Target** | **Step Size** | **Step Delay** |
|---|---|---|
| 55°C | 0.5°C | 5 Cycles |

The amplification scheme generated products of the correct size for K-RAS forward and reverse primers (Seq ID No. 30,61) of the first primer set and the primers of the second primer set (Seq ID No. 63,64). Further optimizations dropped the final total primer concentration down to 0.2uM and decreased the gDNA input from 30- to 10ng. There is no impact on the use of less gDNA on target nucleic acid amplicon quality and the decrease in total primer concentration helped to dissipate but not completely resolve the non-specific bands.

Amplfications using the multi-primer amplification of the first primer set at excesses of 10, 5, 2.5 fold compared to second primer set concentration, equimolar ratio between primer sets and the second primer set in excesses of 2.5, 5 and 10 fold compared to the first primer set concentration generated the first and second amplification products. In every instance the ratios yielded the proper size and products of the primer combination of first and second primer sets. Sizes in relation to 100p ladder run on the gel properly correlate to the predicted sizes of the first and second amplification products shown in Table 4 above. The first amplification product generated by primers with Seq ID No. 30 and 61 is predicted to be 350bp and the second amplification product generated by all four primers (Seq ID No. 30,61,63,64) is predicted to be 420bp.

Further amplification reactions generated PCR products from all 31 first primer set pairs (Tables 1 and 2) in combination with the second primer set pairs (Table 3). The ratio between the concentration of the primers of the first primer set and second primer set was 1:5.

### Example 2

According to the present invention, the Fast Start High Fidelity PCR System, dNTPack (Roche, Catalog No.: 04738292001) was used for amplification in combination with LightCycler® 480 ResoLightDye (Roche, Catalog No.: 04909640001) in order to achieve a reduced incorporation error rate. Similar conditions were used from the initial work with the LightCycler® 480 SYBR Green I Master procedure:

| | |
|---|---|
| FastStart High Fidelity Buffer, 10X Stock without MgCl₂ | 2uL |
| MgCl₂ (25mM Stock) | 2uL |
| PCR Grade Nucleotide Mix | 0.4uL |
| LightCycler® 480 Resolight Dye | 1uL |
| Human genomic DNA (10ng/uL Stock) | 2uL |
| FastStart High Fidelity Enzyme Blend | 0.3uL |
| Primer Mix (0.4uM) | 1.2uL |
| Water, PCR Grade | 11.1uL |
| Total Reaction Volume | 20uL |

PCR Grade Water (Roche, Catalog No.: 03315843001). The FastStart High Fidelity PCR system contains Roche FastStart Taq DNA Polymerase (Roche, Catalog No.: 12032902001) which has 5'-3' polymerase activity with no 3'-5' exonuclease activity and is used in combination with an additional thermostable enzyme with 3'-5' exonuclease (proofreading) activity. Primary amplification reactions used first primer set pairs only at 0.4uM each with MgCl₂ concentrations of 1.8-, 2.4- and 2.8mM.

### Amplification Protocol

| **Step** | | **Temp.** | **Duration** | **Ramp Rate** |
|---|---|---|---|---|
| Initial FastStart Activation | | 95°C | 10min | 4.4°C/sec |
| Amplification (45 Cycles) | | | | |
| | Denaturation | 95°C | 10sec | 4.4°C/sec |
| | Annealing | 63°C | 30sec | 2.2°C/sec |
| | Extension | 72°C | 25sec | 4.4°C/sec |
| Final Extension | | 72°C | 7min | 4.4°C/sec |

| | | | | |
|---|---|---|---|---|
| *Melting Curve program followed amplification* | | | | |

The LightCycler® 480 protocol also used a touch down protocol for optimization and had the following additional settings associated with annealing segment of the Amplification Step:

| **2nd Anneal Target** | **Step Size** | **Step Delay** |
|---|---|---|
| 55°C | 0.5°C | 5 Cycles |

Amplification using the full set of 31 first primer set primer pairs and the second primer set pair produced bands in the expected sizes but only from 28 of the 31 reactions. Optimization conditions included: dropping PCR cycles from 45 to 35, elimination of the final elongation step of 7 minutes and a final primer concentration of 0.2uM. While the FastStart High Fidelity system comprising a 3'-5' thermostable exonuclease activity works in most cases and provides some reduction in non-specific PCR products, further optimization was not pursued.

### Example 3

According to the present invention, experiments performed with the first and second primer sets were also conducted with the Expand High Fidelity System, dNTPack (Roche, Catalog No.: 04738250001) used for amplification in combination with LightCycler® 480 ResoLightDye (Roche, Catalog No.: 04909640001). The reaction mixture for the individual reactions is as follows:

| | |
|---|---|
| Expand High Fidelity Buffer 10X Stock without MgCl₂ | 2uL |
| MgCl₂ (25mM Stock) | 2uL |
| PCR Grade Nucleotide Mix | 0.4uL |
| LightCycler® 480 Resolight Dye | 1uL |
| Human genomic DNA (10ng/uL Stock) | 2uL |
| Expand High Fidelity Enzyme Mix | 0.3uL |
| Primer Mix (0.3 or 0.5uM final Conc.) | 1.2uL |
| Water, PCR Grade | 11.1uL |
| Total Reaction Volume | 20uL |

PCR Grade Water (Roche, Catalog No.: 03315843001). The Expand High Fidelity System contains an enzyme blend which comprises Taq DNA Polymerase and also Tgo DNA Polymerase which, unlike the Taq DNA Polymerase component, has a 3'-5' exonuclease activity (referred to also as "proofreading activity"). The final concentration of primers used was either a total of 0.3uM or 0.5uM. Within these total concentrations were the ratios between first primer set and second primer set concentrations. To illustrate this better, the first primer set will be denoted by "X" and the second primer set will be denoted by "Y". A 1:1, 1:2.5, 1:5, and 1:10 X:Y ratio would result in the following concentrations at 0.5uM total primer, respectively: 0.25:0.25uM, 0.2:0.3uM, 0.1:0.4uM, and 0.05:0.45uM. An X:Y ratio would result in the following concentrations at 0.3uM total primer, respectively: 0.15:0.15uM, 0.12:0.18uM, 0.06:0.24uM and 0.03:0.27uM. These concentrations need to only be reversed in order to get a scheme of 10:1, 5:1, 2.5:1 1 and 1:1 for first primer set concentration excesses over second primer set concentrations.

### Amplification Protocol

| **Step** | | **Temp.** | **Duration** | **Ramp Rate** |
|---|---|---|---|---|
| Pre-incubation | | 95°C | 2min | 4.4°C/sec |
| Amplification I (10 Cycles) | | | | |
| | Denaturation | 95°C | 10sec | 4.4°C/sec |
| | Annealing | 63°C | 30sec | 2.2°C/sec |
| | Extension | 72°C | 30sec | 4.4°C/sec |
| Amplification II (10 Cycles) | | | | |
| | Denaturation | 95°C | 10sec | 4.4°C/sec |
| | Annealing | 59°C | 30sec | 2.2°C/sec |
| | Extension | 72°C | 60sec | 4.4°C/sec |
| Amplification III (10 Cycles) | | | | |
| | Denaturation | 95°C | 10sec | 4.4°C/sec |
| | Annealing | 55°C | 30sec | 2.2°C/sec |
| | Extension | 72°C | 90sec | 4.4°C/sec |
| Amplification IV (5 Cycles) | | | | |
| | Denaturation | 95°C | 10sec | 4.4°C/sec |
| | Annealing | 63°C | 30sec | 2.2°C/sec |
| | Extension | 72°C | 3min | 4.4°C/sec |

| | | | | |
|---|---|---|---|---|
| *Melting Curve program followed amplification* | | | | |

The LightCycler® 480 protocol also used a three different touch down amplification steps during the protocol for optimization and had the following additional settings associated with annealing segment:

### Amplification Step I:

| **2nd Anneal Target** | **Step Size** | **Step Delay** |
|---|---|---|
| 59°C | 0.5°C | 2 Cycles |

| **Amplification Step II:** | | |
|---|---|---|
| **2nd Anneal Target** | **Step Size** | **Step Delay** |
| 55°C | 0.5°C | 0 Cycles |

| **Amplification Step III:** | | |
|---|---|---|
| **2nd Anneal Target** | **Step Size** | **Step Delay** |
| 55°C | 0°C | 0 Cycles |

In these experiments the second primer set pair was used with a 3' phosphate modification and eight different multi-primer sets were chosen. After some optimization steps the PCR products showed clear bands on the gel (increase of specificity) and the PCR also worked with the challenging primers which failed in reactions with the Fast Start High Fidelity system (Example 2). Amplification products generated by the multi-primer approach were from primer pairs Seq ID No. 30, 61; 15,46; 24,55; 25,56; 26,57; 27,58; 28,59; 29,60 of the first primer set (Tables 1 and 2) and Seq ID No. 63,64 of the second primer set (Table 3). For all different ratios of X:Y, the correct second amplification product was obtained.

### Example 4

Further amplifications using three separate amplification systems with modified and unmodified second primer set primers were performed. Results are illustrated in Figure 3 using the multi-primer approach with primers pairs Seq ID No. 5,36,63,64 (300); 15,46,63,64 (310); and 26,57,63,64 (320). The conditions for each set of amplification conditions are as described below.

In Figure 3, represented by each lane containing the letter "A", are the results of the multi-primer approach with the RealTime *ready* DNA Probes Master. RealTime *ready* DNA Probes Master (Roche, Catalog No.: 05502381001) was tested with the multi-primer system without the 3'P-modification in the second primer set pair. The amplification reaction mixture and PCR protocol is listed below.

### Amplification Mixture

| | |
|---|---|
| RealTime ready DNA Probes Master(5X) | 4uL |
| LightCycler 480 Resolight Dye | 1uL |
| Human genomic DNA (10ng/uL Stock) | 2uL |
| Primer Mix | 1.2uL |
| Water, PCR Grade | 11.8uL |
| Total Reaction Volume | 20uL |

PCR Grade Water (Roche, Catalog No.: 03315843001), LightCycler® 480 ResoLightDye (Roche, Catalog No.: 04909640001). The RealTime ready DNA Probes Master contains the AptaTaq DNA Polymerase (Roche, Catalog No.: 05457882103) which does not contain any 3'-5' exonuclease activity. Primers were run at 1:1 first:second primer set ratios.

### Amplification Protocol

| **Step** | | **Temp.** | **Duration** | **Ramp Rate** |
|---|---|---|---|---|
| Pre-Incubation | | 95°C | 1min | 4.4°C/sec |
| Amplification (35 Cycles) | | | | |
| | Denaturation | 95°C | 1sec | 4.4°C/sec |
| | Annealing | 63°C | 30sec | 2.2°C/sec |
| | Extension | 72°C | 25sec | 4.4°C/sec |
| Final Extension | | 72°C | 7min | 4.4°C/sec |

| | | | | |
|---|---|---|---|---|
| *Melting Curve program followed amplification* | | | | |

The LightCycler® 480 protocol also used a touch down protocol and had the following additional settings associated with annealing segment of the Amplification Step:

| **2nd Anneal Target** | **Step Size** | **Step Delay** |
|---|---|---|
| 55°C | 0.5°C | 5 Cycles |

In Figure 3, represented by each lane containing the letter "B", are the results of the multi-primer approach with the FastStart High Fidelity PCR Sytem, dNTPack (Roche, Catalog No.: 04738292001). The amplification reaction mixture and PCR protocol is listed below.

| | |
|---|---|
| FastStart High Fidelity Buffer, 10X Stock without MgCl₂ | 2uL |
| MgCl₂ (25mM Stock) | 2uL |
| PCR Grade Nucleotide Mix | 0.4uL |
| LightCycler® 480 Resolight Dye | 1uL |
| Human genomic DNA (10ng/uL Stock) | 2uL |
| FastStart High Fidelity Enzyme Blend | 0.3uL |
| Primer Mix (0.4uM) | 1.2uL |
| Water, PCR Grade | 11.1uL |
| Total Reaction Volume | 20uL |

PCR Grade Water (Roche, Catalog No.: 03315843001). The FastStart High Fidelity PCR system contains Roche FastStart Taq DNA Polymerase (Roche, Catalog No.: 12032902001) which has 5'-3' polymerase activity with no 3'-5' exonuclease activity but is used in combination with a thermostable enzyme with 3'-5' exonuclease (proofreading) activity.

### Amplification Protocol

| **Step** | | **Temp.** | **Duration** | **Ramp Rate** |
|---|---|---|---|---|
| Initial FastStart Activation | | 95°C | 10min | 4.4°C/sec |
| Amplification (35 Cycles) | | | | |
| | Denaturation | 95°C | 10sec | 4.4°C/sec |
| | Annealing | 63°C | 30sec | 2.2°C/sec |
| | Extension | 72°C | 25sec | 4.4°C/sec |
| Final Extension | | 72°C | 7min | 4.4°C/sec |

| | | | | |
|---|---|---|---|---|
| *Melting Curve program followed amplification* | | | | |

The LightCycler® 480 protocol also used a touch down protocol for optimization and had the following additional settings associated with annealing segment of the Amplification Step:

| **2nd Anneal Target** | **Step Size** | **Step Delay** |
|---|---|---|
| 55°C | 0.5°C | 5 Cycles |

In Figure 3, represented by each lane containing the letter "C", are the results of the multi-primer approach with the Expand High Fidelity System, dNTPack (Roche, Catalog No.: 04738250001) using unmodified second primer set primers. In each lane containing the letter "D" are the results of the multi-primer approach with the Expand High Fidelity System, dNTPack (Roche, Catalog No.: 04738250001) using 3'P-modified second primer set primers. PCR conditions were as disclosed in Example 3.

The results of the RealTime *ready* DNA Probes Master showed products of the correct sizes as seen on the agarose gel, but there were also non-specific smears and primer dimers in each case. The FastStart High Fidelity system reactions using the multi-primer approach without the 3'-P modification of the second primer pairs also produced smearing and low molecular weight amplification products with additional low product yield as compared to other systems tested.

It is obvious from the gel profile that the Expand High Fidelity System with the 3' Phosphate modified second primer set primers generated the proper first and second amplification products and also contained the least amount of non-specific products and high molecular smearing. This is also conserved when amplified according to a multi-primer scheme wherein the first primer set primer are in 5-fold excess over the concentration of the primers of the second primer set.

As can be seen in Figure 3, the unmodified second primer set pairs did not work as well in the Expand High Fidelity system as compared to those that were at least partially modified by a phosphate group at their 3' terminus. With the Expand High Fidelity system in combination with the 3'P-modified second primer set pair, the highest specificity was obtained.

Sequencing of some PCR products of the Expand High Fidelity System reactions was performed using the 5' portion of one of the second primer set primers as a sequencing primer. The PCR products were purified using the High Pure PCR Product Purification Kit (Roche, Catalog No.:11732668001) according to the instruction manual from one amplification using the first primer set pair with genomic DNA as template DNA. For the hg DNA, one sample contained unmodified primer amplified products and the other contained 3'P modified second primer set pairs. All samples could be sequenced corroborating the proper generation of amplification products by sequencing and not just by the distance run on an agarose gel compared to a ladder. It is clear to one of ordinary skill in the art that sequencing can only be successful if the long PCR product was made.

## Claims

1. A method for amplifying target nucleic acids comprising:
(a) providing a first primer set with a forward and reverse primer such that each primer contains a target specific region capable of hybridizing to a target nucleic acid, wherein said forward primer comprises a first tag sequence at its 5' end and said reverse primer comprises a second tag sequence at its 5' end;
(b) providing a second primer set comprising a forward primer with a 3' region identical to the first tag sequence at the 5' end of said forward primer of the first primer set and said reverse primer with a 3' region identical to the second tag sequence at the 5' end of the reverse primer of the first primer set;
(c) amplifying the target nucleic acids in the presence of said first and second primer sets wherein the polymerase has proofreading activity or an enzyme mixture that combines a polymerase lacking proofreading activity with a 3'-5' thermostable exonuclease; and
thereby amplifying multiple target nucleic acids.

2. The method according to claim 1, wherein at least one of the primers of the second primer set are at least partially modified at the 3' terminal nucleotide by a phosphate.

3. The method according to claims 1-2, wherein the primers of the first primer set are at least a 5-fold excess of the primers of the second primer set.

4. The method according to claims 1-3, wherein multiple amplification mixtures are used to amplify multiple target nucleic acids such that the forward primers of the first primer set comprise identical first tag sequences with each other and the reverse primers of the first primer set comprise identical second tag sequences.

5. The method according to claims 1-4, wherein multiple amplification mixtures are used to amplify multiple target nucleic acids such that the forward primers of the first primer set comprise first tag sequences which are different from each other and the reverse primers of the first primer set comprise second tag sequences which are different from each other.

6. The method according to claims 1-5, wherein one of the additional sequences of the primers of the second primer set is a sequence used for identification of the target nucleic acid amplicons.

7. The method according to claims 1-6, wherein one of the additional sequences of the primers of the second primer set is a sequence used for an amplification primer site.

8. The method according to claims 1-7, wherein one of the additional sequences of the primers of the second primer set is a sequence used for a sequencing primer site.

9. The method according to claims 1-8, wherein the target nucleic acid amplicons are at least 300 base pairs in length.

10. The method according to claims 1-9, wherein the primers of the first primer set are at a 5-fold excess in concentration over the concentration of the primers of the second primer set.

11. A kit for amplifying target nucleic acids comprising:
(a) a first primer set with a at least one forward and reverse primer pair such that each primer contains a target specific region capable of hybridizing to a target nucleic acid, wherein said forward primer comprises a first tag sequence at its 5' end and said reverse primer comprises a second tag sequence at its 5' end;
(b) a second primer set comprising a forward primer with a 3' region identical to the first tag sequence at the 5' end of said forward primer of the first primer set and said reverse primer with a 3' region identical to the second tag sequence at the 5' end of the reverse primer of the first primer set; and
(c) a polymerase with proofreading activity or an enzyme mixture that combines a polymerase lacking proofreading activity with a 3'-5' thermostable exonuclease.

12. A kit according to claim 11, wherein the forward primers of the first primer set comprise identical first tag sequences with each other and the reverse primers of the first primer set comprise identical second tag sequences.

13. A kit according to claims 11-12, the forward primers of the first primer set comprise first tag sequences which are different from each other and the reverse primers of the first primer set comprise second tag sequences which are different from each other.

14. A kit according to claims 11-13, wherein one of the additional sequences of the primers of the second primer set is a sequence used for identification of the target nucleic acid amplicons.

15. A kit according to claims 11-14, wherein the additional sequences of the primers of the second primer set includes a sequence used for an amplification primer and a sequencing primer site.


SEQUENCE LISTING

<110>  Roche Diagnostics GmbH, Mannheim (DE)
       F. Hoffmann - La Roche AG, Basel (CH)

<120>  Improved Method for Amplification of Target Nucleic Acids Using a
        Multi-Primer Approach

<130>  30595 EP1

<150>  EP 11177735.5
<151>  2011-08-17

<160>  64

<170>  PatentIn version 3.5

<210>  1
<211>  42
<212>  DNA
<213>  Artificial Sequence

<220>
<223>  Primer Sequence

<400>  1
gttgtaaaac gacggccagt attcaactag agggcagcct tg                          42


<210>  2
<211>  43
<212>  DNA
<213>  Artificial Sequence

<220>
<223>  Primer Sequence

<400>  2
gttgtaaaac gacggccagt gaataccctg tatgaaggga agc                         43


<210>  3
<211>  40
<212>  DNA
<213>  Artificial Sequence

<220>
<223>  Primer Sequence

<400>  3
gttgtaaaac gacggccagt tgtagcccaa gaaaatgcag                             40


<210>  4
<211>  44
<212>  DNA
<213>  Artificial Sequence

<220>
<223>  Primer Sequence

<400>  4
gttgtaaaac gacggccagt catctcacat aaatgccatt aaca                        44

<210>  5
<211>  42
<212>  DNA
<213>  Artificial Sequence

<220>
<223>  Primer Sequence

<400>  5
gttgtaaaac gacggccagt gaaaataaca tccagggaac ca                          42


<210>  6
<211>  43
<212>  DNA
<213>  Artificial Sequence

<220>
<223>  Primer Sequence

<400>  6
gttgtaaaac gacggccagt ggagttttag aagaacacca cca                         43


<210>  7
<211>  41
<212>  DNA
<213>  Artificial Sequence

<220>
<223>  Primer Sequence

<400>  7
gttgtaaaac gacggccagt ccaatttttg gtagcagtgg a                           41


<210>  8
<211>  42
<212>  DNA
<213>  Artificial Sequence

<220>
<223>  Primer Sequence

<400>  8
gttgtaaaac gacggccagt tgacctccaa acaatacact gg                          42


<210>  9
<211>  38
<212>  DNA
<213>  Artificial Sequence

<220>
<223>  Primer Sequence

<400>  9
gttgtaaaac gacggccagt cccagtgttg aaacagca                               38


<210>  10
<211>  45
<212>  DNA
<213>  Artificial Sequence

<220>
<223>  Primer Sequence

<400>  10
gttgtaaaac gacggccagt tggtgaaaat cagtattcaa aatca                    45


<210>  11
<211>  42
<212>  DNA
<213>  Artificial Sequence

<220>
<223>  Primer Sequence

<400>  11
gttgtaaaac gacggccagt cttcttcaca ggtgctttca ag                       42


<210>  12
<211>  41
<212>  DNA
<213>  Artificial Sequence

<220>
<223>  Primer Sequence

<400>  12
gttgtaaaac gacggccagt ttgccatagt cagatgcaca g                        41


<210>  13
<211>  42
<212>  DNA
<213>  Artificial Sequence

<220>
<223>  Primer Sequence

<400>  13
gttgtaaaac gacggccagt ttgactagac aaaccactgc tg                       42


<210>  14
<211>  40
<212>  DNA
<213>  Artificial Sequence

<220>
<223>  Primer Sequence

<400>  14
gttgtaaaac gacggccagt ggggttaagc tttgtggatg                          40


<210>  15
<211>  42
<212>  DNA
<213>  Artificial Sequence

<220>
<223>  Primer Sequence

<400>  15
gttgtaaaac gacggccagt aaaccgttca tttctcagga tg                       42

<210>  16
<211>  40
<212>  DNA
<213>  Artificial Sequence

<220>
<223>  Primer Sequence

<400>  16
gttgtaaaac gacggccagt tgcaagtgac ccttgttttg                              40


<210>  17
<211>  46
<212>  DNA
<213>  Artificial Sequence

<220>
<223>  Primer Sequence

<400>  17
gttgtaaaac gacggccagt atagacacct ataatatcag ctgcac                       46


<210>  18
<211>  44
<212>  DNA
<213>  Artificial Sequence

<220>
<223>  Primer Sequence

<400>  18
gttgtaaaac gacggccagt ccatatattg tgtttgggat tcaa                         44


<210>  19
<211>  44
<212>  DNA
<213>  Artificial Sequence

<220>
<223>  Primer Sequence

<400>  19
gttgtaaaac gacggccagt tgctctattt tgtgtcattc catt                         44


<210>  20
<211>  41
<212>  DNA
<213>  Artificial Sequence

<220>
<223>  Primer Sequence

<400>  20
gttgtaaaac gacggccagt gggacctgta gttgaggctg t                            41


<210>  21
<211>  40
<212>  DNA

<213>  Artificial Sequence

<220>
<223>  Primer Sequence

<400>  21
gttgtaaaac gacggccagt gagtttggga gtgtggaagc                              40


<210>  22
<211>  44
<212>  DNA
<213>  Artificial Sequence

<220>
<223>  Primer Sequence

<400>  22
gttgtaaaac gacggccagt gccttcataa aataatcatc aaca                         44


<210>  23
<211>  43
<212>  DNA
<213>  Artificial Sequence

<220>
<223>  Primer Sequence

<400>  23
gttgtaaaac gacggccagt ccaatccagt tagtccttat cca                          43


<210>  24
<211>  40
<212>  DNA
<213>  Artificial Sequence

<220>
<223>  Primer Sequence

<400>  24
gttgtaaaac gacggccagt gctcagtcta ccacccatcc                              40


<210>  25
<211>  43
<212>  DNA
<213>  Artificial Sequence

<220>
<223>  Primer Sequence

<400>  25
gttgtaaaac gacggccagt cattcacctt ctcacataat cca                          43


<210>  26
<211>  40
<212>  DNA
<213>  Artificial Sequence

<220>
<223>  Primer Sequence

<400>  26
gttgtaaaac gacggccagt agacagcgag cagagctttc                              40


<210>  27
<211>  40
<212>  DNA
<213>  Artificial Sequence

<220>
<223>  Primer Sequence

<400>  27
gttgtaaaac gacggccagt agcccgtgag aaagaggaag                              40


<210>  28
<211>  44
<212>  DNA
<213>  Artificial Sequence

<220>
<223>  Primer Sequence

<400>  28
gttgtaaaac gacggccagt ccagactaga tgctttctgg ttta                         44


<210>  29
<211>  44
<212>  DNA
<213>  Artificial Sequence

<220>
<223>  Primer Sequence

<400>  29
gttgtaaaac gacggccagt gatgcatctg ttactatctt ttgc                         44


<210>  30
<211>  45
<212>  DNA
<213>  Artificial Sequence

<220>
<223>  Primer Sequence

<400>  30
gttgtaaaac gacggccagt ggtactggtg gagtatttga tagtg                        45


<210>  31
<211>  46
<212>  DNA
<213>  Artificial Sequence

<220>
<223>  Primer Sequence

<400>  31
gttgtaaaac gacggccagt tgtaataatc cagactgtgt ttctcc                       46


<210>  32

<211>  42
<212>  DNA
<213>  Artificial Sequence

<220>
<223>  Primer Sequence

<400>  32
cacaggaaac agctatgacc actgtgcgtt ttattcctcc at                          42


<210>  33
<211>  42
<212>  DNA
<213>  Artificial Sequence

<220>
<223>  Primer Sequence

<400>  33
cacaggaaac agctatgacc cccactgcag ttatgtgttg aa                          42


<210>  34
<211>  40
<212>  DNA
<213>  Artificial Sequence

<220>
<223>  Primer Sequence

<400>  34
cacaggaaac agctatgacc tgggtgagtg atctcacagg                             40


<210>  35
<211>  39
<212>  DNA
<213>  Artificial Sequence

<220>
<223>  Primer Sequence

<400>  35
cacaggaaac agctatgacc agcttgcaaa ttgctgctg                              39


<210>  36
<211>  44
<212>  DNA
<213>  Artificial Sequence

<220>
<223>  Primer Sequence

<400>  36
cacaggaaac agctatgacc ccctctattt tcacttccct taaa                        44


<210>  37
<211>  41
<212>  DNA
<213>  Artificial Sequence

<220>

<223>  Primer Sequence

<400>  37
cacaggaaac agctatgacc tcgacccttc agaatctctt g                41


<210>  38
<211>  40
<212>  DNA
<213>  Artificial Sequence

<220>
<223>  Primer Sequence

<400>  38
cacaggaaac agctatgacc cttgtgctcc agctgtgttg                40


<210>  39
<211>  42
<212>  DNA
<213>  Artificial Sequence

<220>
<223>  Primer Sequence

<400>  39
cacaggaaac agctatgacc tgagtttgaa aatggctcag tc                42


<210>  40
<211>  45
<212>  DNA
<213>  Artificial Sequence

<220>
<223>  Primer Sequence

<400>  40
cacaggaaac agctatgacc ttatattctg tacttcctcc agtcc                45


<210>  41
<211>  43
<212>  DNA
<213>  Artificial Sequence

<220>
<223>  Primer Sequence

<400>  41
cacaggaaac agctatgacc ccctgtagaa ctgaagcttg ttg                43


<210>  42
<211>  39
<212>  DNA
<213>  Artificial Sequence

<220>
<223>  Primer Sequence

<400>  42
cacaggaaac agctatgacc atacaggcat gtggcttgc                39

<210>  43
<211>  43
<212>  DNA
<213>  Artificial Sequence

<220>
<223>  Primer Sequence

<400>  43
cacaggaaac agctatgacc ctgaagaagt tgtttgctgc tct                43


<210>  44
<211>  44
<212>  DNA
<213>  Artificial Sequence

<220>
<223>  Primer Sequence

<400>  44
cacaggaaac agctatgacc tttatgagcc tttacaaatt gctg               44


<210>  45
<211>  43
<212>  DNA
<213>  Artificial Sequence

<220>
<223>  Primer Sequence

<400>  45
cacaggaaac agctatgacc ttgtgactct ctggtgaata gca                43


<210>  46
<211>  45
<212>  DNA
<213>  Artificial Sequence

<220>
<223>  Primer Sequence

<400>  46
cacaggaaac agctatgacc gtaatgttct ttttaactgg catga              45


<210>  47
<211>  40
<212>  DNA
<213>  Artificial Sequence

<220>
<223>  Primer Sequence

<400>  47
cacaggaaac agctatgacc aaccaaagat tgggctttcc                    40


<210>  48
<211>  43
<212>  DNA
<213>  Artificial Sequence

<220>
<223>  Primer Sequence

<400>  48
cacaggaaac agctatgacc cagtttggga aaaactttga tta                         43


<210>  49
<211>  43
<212>  DNA
<213>  Artificial Sequence

<220>
<223>  Primer Sequence

<400>  49
cacaggaaac agctatgacc gcagtggttt caacaattaa gag                         43


<210>  50
<211>  43
<212>  DNA
<213>  Artificial Sequence

<220>
<223>  Primer Sequence

<400>  50
cacaggaaac agctatgacc cagtgtgaga acagactcaa cag                         43


<210>  51
<211>  40
<212>  DNA
<213>  Artificial Sequence

<220>
<223>  Primer Sequence

<400>  51
cacaggaaac agctatgacc ggggctgact tttccttttc                             40


<210>  52
<211>  40
<212>  DNA
<213>  Artificial Sequence

<220>
<223>  Primer Sequence

<400>  52
cacaggaaac agctatgacc gggggcaaaa ccaaaataat                             40


<210>  53
<211>  40
<212>  DNA
<213>  Artificial Sequence

<220>
<223>  Primer Sequence

<400>  53

cacaggaaac agctatgacc ctgcagcttg agatgaggtg                                40

<210>  54
<211>  43
<212>  DNA
<213>  Artificial Sequence

<220>
<223>  Primer Sequence

<400>  54
cacaggaaac agctatgacc aaaactctgg ctatttccaa acc                           43

<210>  55
<211>  40
<212>  DNA
<213>  Artificial Sequence

<220>
<223>  Primer Sequence

<400>  55
cacaggaaac agctatgacc agatgcaggg catgaagaga                                40

<210>  56
<211>  41
<212>  DNA
<213>  Artificial Sequence

<220>
<223>  Primer Sequence

<400>  56
cacaggaaac agctatgacc gaattgaccc atgagttgga g                              41

<210>  57
<211>  40
<212>  DNA
<213>  Artificial Sequence

<220>
<223>  Primer Sequence

<400>  57
cacaggaaac agctatgacc aagtttcatg tggctcagca                                40

<210>  58
<211>  40
<212>  DNA
<213>  Artificial Sequence

<220>
<223>  Primer Sequence

<400>  58
cacaggaaac agctatgacc actgtgacct ttccccactg                                40

<210>  59
<211>  43

<212>  DNA
<213>  Artificial Sequence

<220>
<223>  Primer Sequence

<400>  59
cacaggaaac agctatgacc ccgaattttc caaggttatt aca                    43


<210>  60
<211>  45
<212>  DNA
<213>  Artificial Sequence

<220>
<223>  Primer Sequence

<400>  60
cacaggaaac agctatgacc cctatcattg tagtttggat atcgt                  45


<210>  61
<211>  42
<212>  DNA
<213>  Artificial Sequence

<220>
<223>  Primer Sequence

<400>  61
cacaggaaac agctatgacc tgaaacccaa ggtacatttc ag                     42


<210>  62
<211>  46
<212>  DNA
<213>  Artificial Sequence

<220>
<223>  Primer Sequence

<400>  62
cacaggaaac agctatgacc cagggatatt acctacctca taaaca                 46


<210>  63
<211>  55
<212>  DNA
<213>  Artificial Sequence

<220>
<223>  Primer Sequence

<400>  63
cgtatcgcct ccctcgcgcc atcagacgag tgcgtgttgt aaaacgacgg ccagt       55


<210>  64
<211>  55
<212>  DNA
<213>  Artificial Sequence

<220>
<223>  Primer Sequence

<400> 64
ctatgcgcct tgccagcccg ctcagacgag tgcgtcacag gaaacagcta tgacc 55